# EUROPEAN PATENT APPLICATION

(11) **EP 1 020 435 A1**
(43) Date of publication of application: **19.07.2000**
(21) Application number: 98900367.8
(22) Date of filing: 14.01.1998
(51) Int. Cl.: C07C 265/04, C07C 263/10, C08G 18/73, C09D 175/04, C09J 175/04

(54) **ALIPHATIC TRIISOCYANATE COMPOUND, PROCESS FOR PRODUCING THE SAME, AND POLYURETHANE RESIN MADE FROM THE COMPOUND**

(30) Priority: 16.01.1997 JP 580097
(71) Applicant: Nippon Kasei Chemical Company Limited, Iwaki-shi Fukushima-ken, 971-8101 (JP)
(72) Inventor: KITAI, M., Kurosaki Plant, Mitsubishi Chemical, Kitakyushu-shi, Fukuoka 806 (JP); RYUUTOU, H., Kurosaki Plant, Mitsubishi Chemical, Kitakyushu-shi, Fukuoka 806 (JP); YAHATA, T., Kurosaki Plant, Mitsubishi Chemical, Kitakyushu-shi, Fukuoka 806 (JP); HARA, Yoshinori, Yokohama Res. Center, Aoba-ku, Yokohama-shi, Kanagawa 227 (JP); IWANE, Hiroshi, Tsukuba Res. Center, Ami-machi, Inashiki-gun, Ibaraki 300-03 (JP); SOEJIMA, Yuuji, Yokohama Res. Center, Aoba-ku, Yokohama-shi, Kanagawa 227 (JP)
(74) Representative: HOFFMANN - EITLE
(86) International application number: JP9800115
(87) International publication number: WO9831662

(57) **Abstract**

3-isocyanate methyl-1,6-hexamethylene diisocyanate which is useful for the production of polyurethane resins such as polyurethane polyisocyanate; a process for producing 3-isocyanate methyl-1,6-hexamethylene diisocyanate which process comprises reacting 3-aminomethyl-1,6-hexamethylene diamine with phosgene in the presence of a tertiary amine; and a paint or an adhesive containing the polyurethane resin produced using the above isocyanate compound.

## Description

### Technical Field

The present invention relates to a novel aliphatic triisocyanate compound and a process for producing the compound. Further, the present invention also relates to a polyurethane resin produced by using the isocyanate compound, and a paint, an adhesive, etc. containing the polyurethane resin.

### Background Art

Polyurethane resins which are produced by reacting an isocyanate compound with a polyol compound at a reaction ratio (molar ratio) of "(isocyanate group/active hydrogen group (hydroxy group, etc.))" of more than 1, and which have isocyanate groups bonded to molecular ends thereof (in the present specification, occasionally referred to as "polyurethane polyisocyanate"), have been used as a curing agent for resins containing an active hydrogen, and has been widely applied to, for example, paints for metals, plastics, woods, magnetic recording media, etc., adhesives or the like. A dryability and a curability of a coating film and properties of the coating film are important factors of good paints or adhesives. The number of functional groups and the reactivity of isocyanate groups in the polyurethane polyisocyanate contribute to these properties to a large extent.

Hitherto, in the production of yellowing-free polyurethane polyisocyanates, there have been used organic diisocyanate compounds such as hexamethylene diisocyanate, isophorone diisocyanate or the like. In addition, as polyurethane polyisocyanates which are increased in number of functional groups, there have been used reaction products obtained from these organic diisocyanate compounds and short-chain polyol compounds, polyisocyanurate derivatives, of the organic diisocyanate compounds, or the like. However, since these polyurethane polyisocyanates have a high viscosity and, therefore, are difficult to handle, it is usually required to dilute these compounds with a solvent upon the use thereof.

Polyurethane resins having hydroxy groups bonded to molecular ends thereof, which are produced by reacting an isocyanate compound with an excessive amount of a polyol compound, etc., at such a ratio of "(isocyanate group/active hydrogen group)" of less than 1 so as to (in the present specification, occasionally referred to as "polyurethane polyol"), are excellent in wear resistance, flexibility, strength, adhesion property and the like. Therefore, such polyurethane resins have been widely applied to various fields such as paints, ink, adhesives, synthetic leathers and the like. In particular, in the fields of automobiles, domestic electrical appliances, building materials or the like, there has been an rapidly increasing demand for polyurethane polyols as paints for metals, plastics, woods, etc., so that various polyurethane polyols satisfying respective performance requirements have been proposed.

Conventionally, in order to increase the number of hydroxy functional groups of the polyurethane polyol, there have been used many polyol compounds having three or more functional groups. However, glycerols show a low reactivity with isocyanate groups since the glycerols contain a secondary hydroxy group, so that there has been caused such a problem that the reaction therebetween cannot readily proceed. In addition, trimethylol propane, etc., are solids, so that it is inconvenient to handle these compounds upon the production thereof. Further, in the case where conventional polyurethane polyisocyanates such as reaction products produced from diisocyanate compounds and short-chain polyol compounds in order to increase the number of functional groups, are used, there has been caused such a problem that it becomes difficult to control the cross-linking density and the molecular weight because of the increased number of functional groups.

On the other hand, due to the fact that the isocyanate compounds used for the production of the polyurethane resins are harmful to human bodies, it is desired that a vapor pressure of the isocyanate compounds be low. Hitherto, from the above-mentioned viewpoint, as the low-vapor pressure aliphatic isocyanate compounds, there have been proposed 4-isocyanate methyl-1,8-octamethylene diisocyanate (refer to Japanese Patent Application Laid-Open (KOKAI) Nos. 56-61341(1981) and 60-233044(1985)), 4-isocyanate propyl-1,7-heptamethylene diisocyanate (refer to Japanese Patent Application Laid-Open (KOKAI) No. 2-145556(1990)), 1,6,11-undecamethylene triisocyanate (refer to Japanese Patent Application Laid-Open (KOKAI) Nos. 55-327(1980)), or the like.

However, since the known production process of the isocyanate compounds requires a distillation-purifying step in the course thereof, there arises a problem that when the vapor pressure of the isocyanate compounds is too low, it becomes difficult to produce these isocyanate compounds. Accordingly, it has been demanded to provide isocyanate compounds having a low vapor pressure to such an extent as being harmless to human bodies, and capable of being purified by distillation in an industrial scale.

### Disclosure of the Invention

It is an object of the present invention to provide an isocyanate compound having a low vapor pressure to such an extent as being harmless to human bodies, and capable of being purified by distillation in an industrial scale, and provide a process for producing such an isocyanate compound. It is another object of the present invention to provide a polyurethane resin produced using the above-specified isocyanate compound. More specifically, the object of the present invention is to provide a polyurethane resin suitably used for foams, paints, adhesives, coating compositions, films, elastomers or the like.

In an aspect of the present invention, there is provided as an isocyanate compound 3-isocyanate methyl-1,6-hexamethylene diisocyanate represented by the following formula (I):

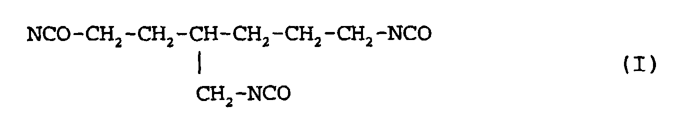

This isocyanate compound has a boiling point of 150°C/2 mmHg, and can be easily vacuum-distilled in an industrial scale. In addition, the isocyanate compound has a vapor pressure of 0.00075 mmHg at a temperature of 25°C. The vapor pressure is as low as about one-twentieth of 0.0150 mmHg which is a vapor pressure of hexamethylene diisocyanate as an extensively used aliphatic isocyanate. Therefore, such isocyanate compound is almost unvaporized at an ordinary temperature.

In an another aspect of the present invention, there is provided a process for producing 3-isocyanate methyl-1,6-hexamethylene diisocyanate, which process comprises reacting 3-aminomethyl-1,6-hexamethylene diamine with phosgene in the presence of a tertiary amine.

This process may comprise reacting 3-aminomethyl-1,6-hexamethylene diamine with phosgene in an inert solvent at a temperature of not more than 40°C in the presence of a tertiary amine so as to produce a slurry containing tertiary amine hydrochloride as a precipitate; heating the slurry to obtain a slurry containing 3-aminomethyl-1,6-hexamethylene diisocyanate; filtering the obtained slurry to separate the slurry into a filter cake composed mainly of the tertiary amine hydrochloride and a filtrate composed mainly of the inert solvent and 3-aminomethyl-1,6-hexamethylene diisocyanate; and then distilling the filtrate in the presence of a hydrochloric acid scavenger.

In an other aspect of the present invention, there is provided a polyurethane resin produced by reacting 3-isocyanate methyl-1,6-hexamethylene diisocyanate with a polyol compound. The polyurethane resin is preferably in the form of polyurethane polyisocyanate or polyurethane polyol. Further, in accordance with the present invention, there is also provided paints or adhesives containing these polyurethane resins.

### Brief Description of Drawings

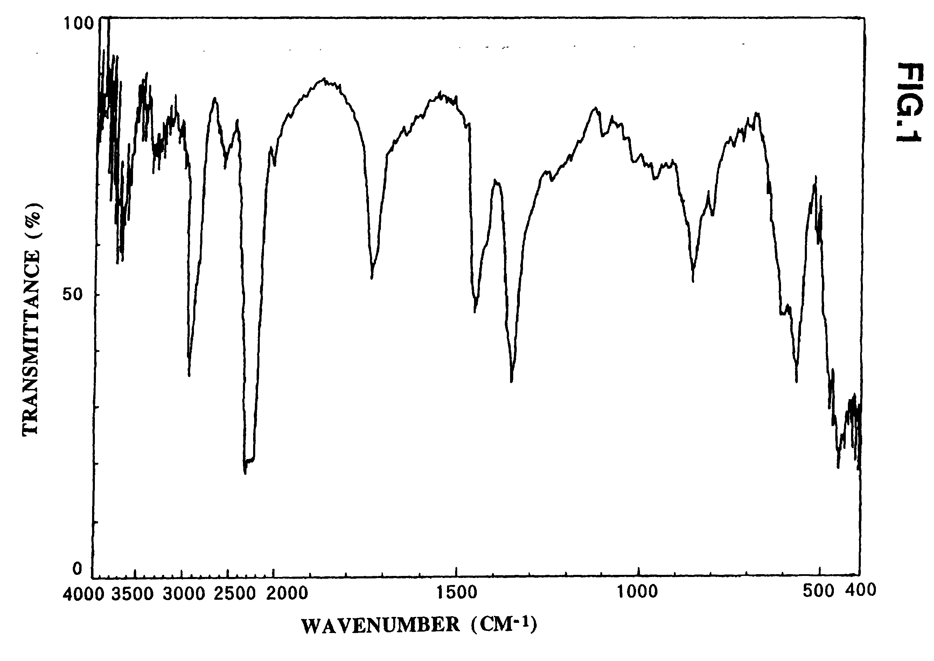

Fig. 1 is a view showing an IR spectrum of 3-isocyanate methyl-1,6-hexamethylene diisocyanate obtained in Example 1.

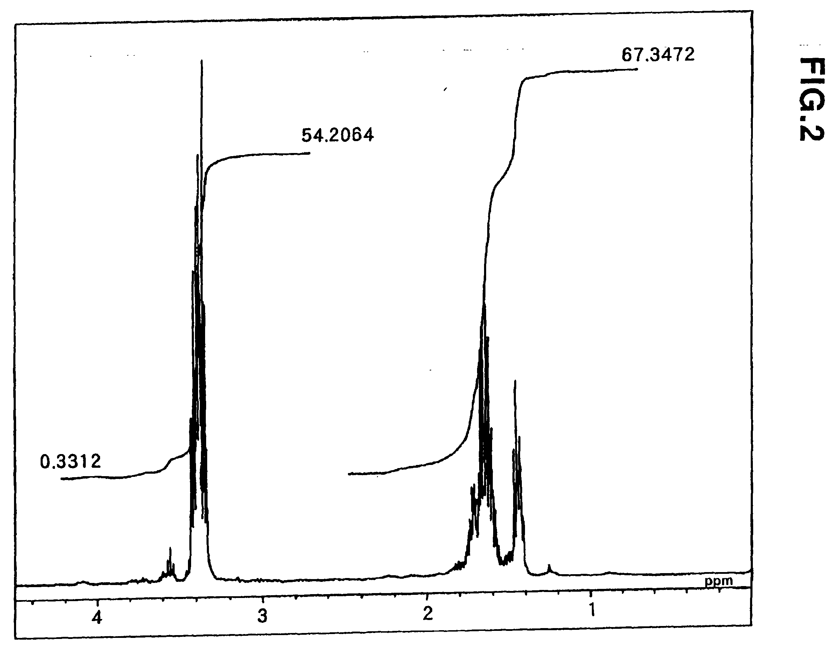

Fig. 2 is a view showing an NMR spectrum of 3-isocyanate methyl-1,6-hexamethylene diisocyanate obtained in Example 1.

### Best Mode for Carrying out the Invention

The 3-isocyanate methyl-1,6-hexamethylene diisocyanate represented by the above formula (I) can be produced by reacting a corresponding triamine, i.e., 3-aminomethyl-1,6-hexamethylene diamine with phosgene by an ordinary method. The 3-aminomethyl-1,6-hexamethylene diamine as a raw material has a boiling point of 122°C/8 mmHg, and can be produced by adding hydrogen cyanide to methylene glutaronitrile obtained by the dimerization of acrylonitrile, and then subjecting the obtained product to hydrogen reduction.

As the method of producing the isocyanate group by reacting amine with phosgene, there can be used the following two methods. One method (1) comprises adding an acid such as hydrochloric acid, sulfuric acid, phosphoric acid, acetic acid or the like to amine so as to obtain an amine acid salt, preferably amine hydrochloride; and then reacting the obtained amine acid salt with phosgene in an inert solvent at a temperature of 60 to 230°C, thereby producing the aimed isocyanate compound. Another method (2) comprises reacting amine with phosgene at a temperature as low as usually not more than 40°C, preferably not more than 10°C to produce carbamoyl chloride; and then subjecting the obtained reaction product to dehydrochlorination at a temperature of 60 to 230°C, preferably 100 to 180°C, thereby producing the aimed isocyanate compound. Both the methods are known to those skilled in the art.

The 3-isocyanate methyl-1,6-hexamethylene diisocyanate according to the present invention can be produced by any of the methods described above. In the former method (1), there can be obtained such an advantage that upon reacting amine with phosgene, the produced isocyanate compound is prevented from reacting with unreacted amine, so that it is possible to avoid the production of a urea compound. However, in this method, since the amine hydrochloride is obtained in the form of coarse particles or aggregates, there is required a bothersome procedure that the hydrochloride should be pulverized before reacting with phosgene. Accordingly, in order to produce the isocyanate compound according to the present invention, in general, the latter method (2) is preferably used.

However, in the latter method (2), as well known in the art, there arises such a problem that when the carbamoyl chloride produced is heated and converted into the isocyanate compound, the carbamoyl chloride tends to be aggregated into masses thereof, and the masses tend to be deposited onto a reactor. The present inventors have found that this problem can be avoided by reacting the amine with phosgene in the presence of a tertiary amine.

More specifically, when 3-aminomethyl-1,6-hexamethylene diamine is reacted with phosgene at a low temperature in a an inert solvent such as benzene, toluene, xylene, monochlorobenzene, o-dichlorobenzene, decalin or the like, a tertiary amine such as pyridine, trimethyl amine, triethyl amine, tributyl amine, quinoline, picoline, N-methyl morpholine, N-ethyl morpholine, pyrazine or the like, are added to the reaction system, so that it is possible to avoid the production and deposition of the aggregates in the subsequent heating step.

The tertiary amine may be preliminarily added to the inert solvent, or may be added to the reaction system together with 3-aminomethyl-1,6-hexamethylene diamine as a raw material. The tertiary amine is reacted with hydrogen chloride produced by the reaction of 3-aminomethyl-1,6-hexamethylene diamine with phosgene, thereby converting into amine hydrochloride. Therefore, the tertiary amine acts as a hydrochloric acid scavenger in the reaction system. The amount of the tertiary amine used is usually not less than 3 moles, preferably about 4 to about 5 moles based on one mole of 3-aminomethyl-1,6-hexamethylene diamine.

Meanwhile, when amine and phosgene are reacted at a low temperature, it is preferred that phosgene always exists in the reaction system in the range of not less than an equivalent amount relative to amino groups contained in the raw material. For this purpose, there may be adopted a method of preliminarily dissolving phosgene in the inert solvent, charging the obtained solution into the reaction system, and then feeding 3-aminomethyl-1,6-hexamethylene diamine as the raw material into the reaction system; or a method of feeding both the components at a molar ratio of phosgene to amine of not less than 3, at the same time.

The reaction of 3-aminomethyl-1,6-hexamethylene diamine with phosgene may be suitably conducted at a temperature of not more than 40°C, preferably not more than 10°C by an ordinary method. Then, the reaction product is heated to a temperature of 40 to 230°C, preferably 100 to 180°C in the presence of phosgene by an ordinary method so as to be subjected to dehydrochlorination reaction, thereby converting the reaction product into 3-isocyanate methyl-1,6-hexamethylene diisocyanate. After completion of the reaction, residual phosgene is removed and the reaction mixture is filtered to remove the tertiary amine hydrochloride suspended therein. Thereafter, the reaction mixture is subjected to distillation, thereby obtaining 3-isocyanate methyl-1,6-hexamethylene diisocyanate. In the distillation step, usually, the reaction mixture is first distilled under from an ordinary pressure to a reduced pressure to remove the inert solvent therefrom, and then the obtained distillation residues are subjected to vacuum distillation so as to distill off the aimed 3-isocyanate methyl-1,6-hexamethylene diisocyanate.

Incidentally, a trace amount of the tertiary amine hydrochloride is still dissolved in a filtrate obtained after filtering off the tertiary amine hydrochloride. In this case, when the filtrate is subjected to distillation, the remaining tertiary amine hydrochloride is dissociated into tertiary amine and acid salts, and the tertiary amine and acid salts are distilled off, got mixed in the aimed product and combined again into tertiary amine hydrochloride, so that the quality of the obtained product tends to be deteriorated. Accordingly, it is preferred that upon distillation of the filtrate, calcium oxide, potassium carbonate, sodium carbonate or the like be added to the filtrate so as to capture hydrochloric acid produced by the dissociation.

The thus obtained 3-isocyanate methyl-1,6-hexamethylene diisocyanate has a boiling point of 150°C/2 mmHg, and is a liquid showing an extremely high fluidity at an ordinary temperature.

Next, the production of the polyurethane resin using the isocyanate compound according to the present invention, is explained.

As polyol compounds used for producing the polyurethane polyisocyanate having terminal isocyanate groups among these polyurethane resins, there may be exemplified polyhydric alcohols having 3 or more functional groups, such as glycerol, trimethylol propane, trimethylol ethane, 1,2,6-hexane triol, 1,2,4-butane triol, erythritol, sorbitol, pentaerythritol, dipentaerythritol or the like; aliphatic glycols such as monomeric glycols, e.g., ethylene glycol, diethylene glycol, propylene glycol, dipropylene glycol, tripropylene glycol, 1,2-butane diol, 1,3-butane diol, 1,4-butane diol, 2,3-butane diol, 2-methyl-1,3-propane diol, 2,3-diethyl-1,3-propane diol, 2-methyl-2-propyl-1,3-propane diol, 2-butyl-2-ethyl-1,3-propane diol, 2,5-pentane diol, 3-methyl-1,5-pentane diol, 2-methyl-2,4-pentane diol, 1,6-hexane diol, 2-ethyl-1,3-hexane diol, neopentyl glycol, 1,3,5-trimethyl-1,3-pentane diol, 2,3,4-trimethyl-1,3-pentane diol, 1,8-octane diol, 1,9-nonane diol, 2-methyl-1,8-octane diol or the like; alicyclic glycols such as 1,4-cyclohexane diol, 1,4-cyclohexane dimethanol or the like; or aromatic glycols such as xylene glycol, bishydroxyethoxy benzene or the like. Further, there may also be used high-molecular weight polyols such as glycols as adducts thereof with bisphenol A ethylene oxide or propylene oxide, polyether polyols, polyester polyols, polyether ester polyols, polycarbonate polyols, polyacrylic polyols or the like.

As the polyether polyols, there may be exemplified glycols such as ethylene glycol, propylene glycol, diethylene glycol or the like; polyols having three or more functional groups, such as glycerol, trimethylol ethane, trimethylol propane, pentaerythritol or the like; hydroxy-containing polyether polyols produced by addition-polymerizing alkylene oxide such as ethylene oxide or propylene oxide with polyamines such as ethylene diamine or triene diamine; polytetramethylene ether glycols obtained by the ring-opening polymerization of tetrahydrofuran; or the like.

As the polyester polyols, there may be exemplified dicarboxylic acids such as succinic acid, adipic acid, sebacic acid, azelaic acid, phthalic acid or the like; tri- or tetracarboxylic acids such as trimellitic acid, pyromellitic acid or the like; diols such as ethylene glycol, propylene glycol, 1,4-butane diol, 1,5-pentane diol, 3-methyl-1,5-pentane diol, 2,2-diethyl propane diol, 2-ethyl-2-butyl propane diol, 1,6-hexane diol, neopentyl glycol, diethylene glycol, 1,4-cyclohexane diol, 1,4-cyclohexane dimethanol or the like; triols such as trimethylol propane, glycerol or the like; polyols obtained by the polycondensation reaction with bisphenol A, bisphenol F, etc.; or the like.

As the polyether ester polyols, there may be exemplified polyether ester polyols obtained by reacting an ether group-containing diol or a mixture of the diol and other glycols with the above-mentioned carboxylic acids or anhydrides of these acids, or by reacting polyester glycol with alkylene oxide, for example, poly(polytetramethylene ether) adipate.

As the polycarbonate polyols, there may be used those polyols obtained by the dealcoholation condensation reaction between polyhydric alcohol and dialkyl carbonate such as dimethyl carbonate or diethyl carbonate; the dephenolation condensation reaction between polyhydric alcohol and diphenyl carbonate; the de-ethyleneglycolation condensation reaction between polyhydric alcohol and ethylene carbonate; or the like. In this case, as the polyhydric alcohols, there may be exemplified aliphatic diols such as 1,6-hexane diol, diethylene glycol, propylene glycol, 1,4-butane diol, 1,5-pentane diol, 3-methyl-1,5-pentane diol, 2,2-diehtyl propane diol, 2-ethyl-2-butyl propane diol, neopentyl glycol, etc.; alicyclic diols such as 1,4-cyclohexane diol, 1,4-cyclohexane dimethanol, etc.; or the like.

Further, there may be used diamines, aminoalcohols or the like. As the diamines, there may be exemplified hexamethylene diamine, xylene diamine, isophorone diamine, N,N-dimethyl ethylene diamine or the like. As the aminoalcohols, there may be exemplified monoethanol amine, diethanol amine or the like.

These polyol compounds suitably have a molecular weight of about 500 to about 5,000. When the polyol compounds having a molecular weight of more than 5,000 are used, there is a tendency that the cross-linking density is decreased, and the strength of coating film is deteriorated.

In the production of the polyurethane resin according to the present invention, other isocyanate compounds can be used together with those of the present invention unless the performance of the obtained polyurethane resin is deteriorated. As such other isocyanate compounds, there may be exemplified aromatic diisocyanates such as carbodiimide-modified compounds or urethoimine-modified compounds of 2,4-tolylene diisocyanate, 2,6-tolylene diisocyanate, xylene-1,4-disocyanate, xylene-1,3-disocyanate, 4,4'-diphenyl methane diisocyanate, 2,4'-diphenyl methane diisocyanate, 4,4'-diphenyl ether diisocyanate, 3,3'-dimethyldiphenyl methane-4,4'-diisocyanate, m-phenylene diisocyanate, p-phenylene diisocyanate, naphthylene-1,4-diisocyanate, naphthylene-1,5-diisocyanate, 3,3'-dimethoxydiphenyl-4,4'-diisocyanate, tetramethyl xylylene diisocyanate, polyphenylene polymethylene polyisocyanate, 4,4'-diphenyl methane diisocyanate, etc.; aliphatic diisocyanates such as tetramethylene diisocyanate, hexamethylene diisocyanate, 2-methyl pentane-1,5-diisocyanate, lysine diisocyanate, etc.; alicyclic diisocyanates such as isophorone diisocyanate, hydrogenated tolylene diisocyanate, hydrogenated xylene diisocyanate, hydrogenated diphenyl methane diisocyanate, etc.; or the like. Further, there may also be used isocyanurate-modified compounds, burette-modified compounds, urethoimine-modified compounds or carbodiimide-modified compounds of these isocyanates, or the like. These isocyanate compounds may be used singly or in the form of a mixture of any two or more thereof. Furthermore, if required, there can be used polyisocyanate compounds having three or more functional groups.

In the production of polyurethane polyisocyanate according to the present invention (urethanation reaction), the reaction temperature used therefor may be selected from the range of usually 10 to 90°C. In the above reaction, no reaction catalyst is usually required. However, in some cases, the use of such catalysts is effective. Examples of the catalysts may include organic tin-based catalysts such as dibutyl tin dilaurate, dibutyl tin dioctoate, etc.; organic lead-based catalysts such as lead octoate, etc.; or the like. Further, tertiary amine-based compounds such as triethyl amine, dimethyloctyl amine, diazabicyclo-undecene or the like can be effectively used as the catalyst. The progress of the above urethanation reaction is followable by measuring the NCO content in the course of the reaction. As a result, the urethanation reaction can be stopped at the time at which the NCO content reaches the aimed value.

The urethanation reaction may be carried out in a solvent. As the solvents used in the urethanation reaction, there may be exemplified aromatic solvents such as toluene, xylene, etc.; ketone-based solvents such as methyl ethyl ketone, methyl isobutyl ketone, cyclohexanone, etc.; ester-based solvents such as ethyl acetate, butyl acetate, isobutyl acetate, etc.; glycol ether ester-based solvents such as ethyleneglycol ethyl ether acetate, propyleneglycol methyl ether acetate, 3-methyl-3-methoxy butyl acetate, ethyl-3-ethoxy propionate, etc.; ether-based solvents such as tetrahydrofuran, dioxane, etc.; or the like. These solvents may be used in the form of a mixture of any two or more thereof. By appropriately selecting the kind, amount and resin concentration of the solvent used, it is possible to control the viscosity of the reaction system according to the conditions upon use.

The polyurethane resins according to the present invention which satisfy the condition as represented by "(isocyanate group/active hydrogen group) > 1" with respect to the functional groups bonded to molecular ends thereof (namely, polyurethane polyisocyanate), have a weight-average molecular weight of 350 to 100,000, preferably 650 to 20,000. When the weight-average molecular weight is more than 100,000, the distance between cross-linked molecules may be disadvantageously increased, so that the strength of coating film tends to be lowered, and the viscosity tends to become too high, thereby sometimes causing the deterioration in workability.

In the case where the polyurethane polyol which is one of the polyurethane resins according to the present invention, is produced by reacting the isocyanate compound of the present invention with the polyol compound, the reaction may be controlled such that the functional groups bonded to the molecular ends of the polyurethane polyol satisfy such a condition as represented by "(isocyanate group/active hydrogen group) < 1". In this case, according to the requirements, there may be optionally used as a terminal stopper, dialkyl amines such as di-n-butyl amine, monoalkyl amines such as butyl amine, monoalcohols such as ethanol, isopropyl alcohol or butanol, monoaminoalcohols such as monoethanol amine or diethanol amine, or the like.

The polyurethane polyols according to the present invention, have a weight-average molecular weight of 5,000 to 300,000, preferably 15,000 to 200,000. When the weight-average molecular weight is less than 5,000, the strength of coating film produced therefrom may be lowered. On the other hand, when the weight-average molecular weight is more than 300,000, the viscosity becomes too high, thereby sometimes causing the deterioration in handling property or workability.

The polyurethane polyols according to the present invention may be optionally blended with one or more kinds of conventional polyurethane polyisocyanates. As the polyurethane polyisocyanates used for this purpose, there may be exemplified trimethylol propane adducts of diisocyanate such as tolylene diisocyanate, diphenylmethane diisocyanate, hexamethylene diisocyanate, isophorone diisocyanate, etc., trimers of the above-mentioned diisocyanates, burette-modified compounds obtained by reacting the above-mentioned diisocyanates with water, or the like.

The paints, coating compositions and adhesives according to the present invention which contain the above-mentioned polyurethane resin, may further contain a polyol compound. As the polyol compounds used for this purpose, there may be suitably used, for example, those having two or more hydroxy groups in a molecule thereof and having a weight-average molecular weight of 50 to 300,000. Specific examples of the polyol compounds may include the above-mentioned monomeric glycols, polyols having three or more functional groups, saturated or unsaturated polyester polyols, saturated or unsaturated oil-modified or fatty acid-modified alkyd polyols, amino-alkyd polyols, polycarbonate polyols, acrylic polyols, polyether polyols, polyester ether polyols, epoxy polyols, polyurethane polyols, cellulose acetate butyrate polyols, fluorine-containing polyols, or the like. Among these polyol compounds, saturated or unsaturated polyester polyols, saturated or unsaturated oil-modified or fatty acid-modified alkyd polyols and acrylic polyols are preferred from the standpoints of film properties (such as gloss, thin film thickness, hardness, flexibility, durability, etc.), workability (dryability, curability, etc.), costs or the like. Incidentally, when the weight-average molecular weight of the polyol compound is more than 300,000, the cross-linking density tends to be lowered and the strength of coating film tends to be deteriorated.

The blending ratio (molar ratio) of the polyurethane polyisocyanate to the polyol compound blended in paints, coating compositions and adhesives according to the present invention, may be adjusted such that the ratio of (isocyanate group/active hydrogen group) in the obtained polyurethane resin is preferably 0.1 to 20, more preferably 0.5 to 15. When the ratio of (isocyanate group/active hydrogen group) in the obtained polyurethane resin is less than 0.1, the cross-linking of the cured product may be insufficient and the film strength may be unsatisfactory, thereby sometimes causing the deterioration in properties such as chemical resistance, solvent resistance or the like. On the other hand, when the ratio of (isocyanate group/active hydrogen group) in the obtained polyurethane resin is more than 20, the cured product becomes brittle, thereby sometimes causing the deterioration in wear resistance.

The paints, coating compositions and adhesives according to the present invention may optionally contain various ordinarily used additives such as a curing acceleration catalyst, a curing retarder, an ultraviolet light absorber, an anti-oxidizing agent, a plasticizer, a leveling agent, etc., and various pigments according to requirements. The paints according to the present invention in which an isocyanate-based curing agent for paints is blended, may be applied by an ordinary coating method. For example, the paints can be applied by an airless sprayer, an air sprayer, electrostatic spray coating, immersion coating, a roll coater, brush, an impact mixing-type sprayer, a paper injection cure (VIC)-type coating machine or the like.

In accordance with the present invention, there is provided polyurethane polyisocyanate which is excellent in reactivity, film properties or the like. In addition, polyurethane-based paints and polyurethane-based adhesives containing the polyurethane resin according to the present invention can show more excellent film properties and adhesion properties than those of conventional paints and adhesives. For this reason, the paints and adhesives according to the present invention can be widely applied to various fields such as paints for metals, plastics, concrete, woods, etc., magnetic recording media such as audio tapes, video tapes, floppy discs, etc., ink, synthetic leathers, adhesives, fibers or the like.

### Examples

The present invention will be described in more detail below by examples. However, these examples are not intended to limit the scope of the present invention. The "part" and "%" in Examples and Comparative Examples represent "part by weight" and "% by weight", respectively, unless otherwise specified.

### Example 1:

530 g of ortho-dichlorobenzene and 98 g of pyridine were charged into a glass reactor equipped with a stirrer, a thermometer, a condenser and a gas-blowing tube, and cooled to 10°C. Successively, 125 g of a phosgene gas was fed into the reaction system and dissolved therein. While maintaining the obtained solution at a temperature of not more than 10°C, a solution obtained by dissolving 40 g of 3-aminomethyl-1,6-hexamethylene diamine in 100 g of ortho-dichlorobenzene, was dropped thereinto for 2 hours under stirring. After completion of the dropping, the stirring of the obtained reaction mixture was further continued for 2 hours. The obtained reaction system was in the form of a slurry containing pyridine hydrochloride and having a relatively high fluidity. While feeding phosgene into the slurry at a feed rate of 30 g/hr, the slurry was heated to 130°C for about 2 hours. At that temperature, phosgene was further fed into the slurry at a feed rate of 30 g/hr for 5 hours. It was confirmed that the reaction system was finally a slurry containing small particles composed of pyridine hydrochloride.

A nitrogen gas was blown into the obtained reaction solution to remove residual phosgene therefrom, and then the reaction solution was filtered to remove the pyridine hydrochloride. The obtained filtrate was subjected to distillation under a pressure of 100 mmHg to distill off ortho-dichlorobenzene. The degree of vacuum in the reaction system was further increased, thereby obtaining 40 g of a distillate having a boiling point of 150°C/ 2 mmHg. As a result of the analysis, the obtained distillate was determined to be 3-isocyanate methyl-1,6-hexamethylene diisocyanate.
Gas chromatography/mass spectrometry (GC-Ms): "M⁺/e = 223" and three NCO groups were determined (molecular weight of 3-isocyanate methyl-1,6-hexamethylene diisocyanate: 223.23).
IR: an extremely strong characteristic absorption based on isocyanate groups was observed at about 2300 cm⁻¹.
¹H-NMR: 7H (CH and three kinds of CH₂) at δₚₚₘ = 1.4 to 1.8 and 6H (three kinds of CH₂) at about 3.4 were determined.
NCO content: the NCO content was measured by the following method, and the results are shown in Table 1. (calculated value: 56.5 %)

### Comparative Example 1:

The viscosity and the NCO content of 1,6-hexamethylene diisocyanate (tradename: DURANAT 50 M (HDI) produced by Asahi Kasei Co., Ltd.) were measured. The results are shown in Table 1.

### Comparative Example 2:

The viscosity and the NCO content of isophorone diisocyanate (tradename: VESTANAT IPDI produced by Hulse Co., Ltd.) were measured. The results are shown in Table 1.

### Comparative Example 3:

The viscosity and the NCO content of DURANAT THA-100 (produced by Asahi Kasei Co., Ltd.; polyisocyanurate derivative of 1,6-hexamethylene diisocyanate (trimer); resin solid content: 100 %) were measured. The results are shown in Table 1.

### Comparative Example 4:

The viscosity and the NCO content of VESTANAT T1890/100 (produced by Hulse Co., Ltd.; polyisocyanurate derivative of isophorone diisocyanate (trimer); resin solid content: 100 %) were measured. The results are shown in Table 1.

### 〈Viscosity measuring method〉

The viscosity of each isocyanate compound was measured according to JIS K-1603 using an E-type viscometer (VISCONIC EHD-R Model, manufactured by Tokimec Co., Ltd.). The measuring temperature was 25°C; the amount of a sample tested was 1.5 ml; and the measurement was conducted using a standard rotor (1°34').

### 〈NCO content measuring method〉

The NCO content of each isocyanate compound was measured according to JIS K-1603.

**Table 1**

| | Kind of isocyanate | Viscosity (mPa.s/25°C) | NCO content (wt %) |
|---|---|---|---|
| Example 1 | 3-isocyanate-1,5-hexamethylene diisocyanate | 10 | 55.4 |
| Comparative Example 1 | 1,6-hexamethylene diisocyanate | 3 | 49.4 |
| Comparative Example 2 | isophorone diisocyanate | 13 | 37.8 |
| Comparative Example 3 | DURANAT THA-100 | 2340 | 21.3 |
| Comparative Example 4 | VESTANAT T1890 | Solid | 16.9 |

### Example 2:

Using 3-isocyanate methyl-1,6-hexamethylene diisocyanate obtained in Example 1 and polyol (tradename: KURARE POLYOL P-1010, hydroxyl value: 116.5 KOH mg/g, produced by Kurare Co., Ltd.), the following evaluation tests were conducted. The results are shown in Table 2.

### Comparative Example 5:

Using 1,6-hexamethylene diisocyanate and the polyol, the following evaluation tests were conducted. The results are shown in Table 2.

### Comparative Example 6:

Using isophorone diisocyanate and the polyol, the following evaluation tests were conducted. The results are shown in Table 2.

### Comparative Example 7:

Using DURANAT THA-100 and the polyol, the following evaluation tests were conducted. The results are shown in Table 2.

### Comparative Example 8:

Using VESTANAT T1890/100 and the polyol, the following evaluation tests were conducted. The results are shown in Table 2.

### 〈Evaluation test〉

### (1) Reactivity:

The isocyanate compound and the polyol compound were mixed together such that the ratio of the number of isocyanate groups to that of hydroxy groups was 1.1:1. The obtained mixture was applied onto a 2 mm-thick glass plate and allowed to stand at 23°C for 5 minutes, thereby forming a coating film having a dry thickness of 3 µm. Thereafter, the obtained coating film was interposed between the glass plate and another glass plate, and the absorbence of isocyanate groups at 2270 cm⁻¹ was measured by an Infrared absorption spectroscope (FT-IR H-230, manufactured by Nippon Bunko Co., Ltd.). Subsequently, the absorbences after heating at 80°C for one hour and 4 hours, were measured respectively. The reactivities (%) were calculated from the rates of reduction in absorbence of isocyanate groups after one hour and 4 hours, respectively, assuming that the absorbence at 23°C after 5 minutes was 100.

### (2) Breaking strength and breaking elongation:

The isocyanate compound and the polyol compound were mixed together such that the ratio of the number of isocyanate groups to that of hydroxy groups was 1.1:1. The obtained mixture was applied onto a release paper, heated at 80°C for 24 hours and then cured, thereby obtaining a coating film having a thickness of 100 µm.

Properties of the obtained coating film were measured at a temperature of 23°C and a relative humidity of 65 % according to JIS K 6301 using TENSILON UTM-III-100 (manufactured by Toyo Bawldwin Co., Ltd.).

### (3) Solvent resistance:

The isocyanate compound and the polyol compound were mixed together such that the ratio of the number of isocyanate groups to that of hydroxy groups was 1.1:1. The obtained mixture was applied onto a glass plate, heated at 80°C for 24 hours and then cured, thereby obtaining a coating film having a thickness of 100 µm.

A solvent shown in Table 2 was topped onto the surface of the obtained coating film. 30 seconds after completion of the topping, the solvent was swept, and the surface condition of the coating film was visually observed and evaluated according to the following criteria:
- ⓞ:: No change was recognized.
- ○:: Contour of the solvent remained.
- △:: A part of the coating film was dissolved.
- X:: A greater part of the coating film was dissolved.

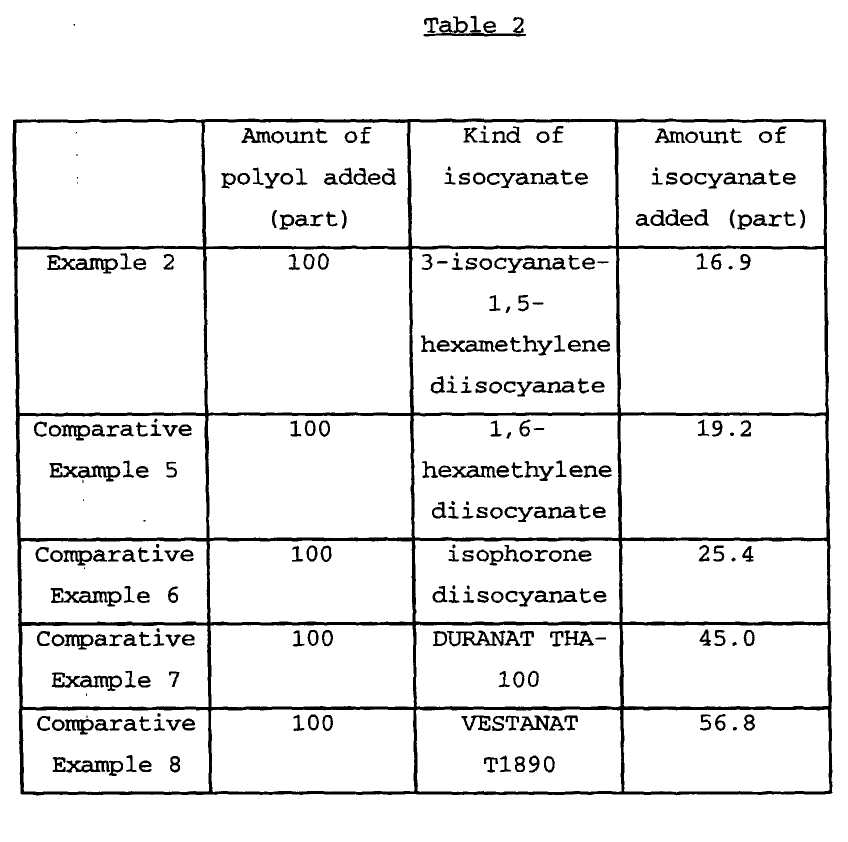

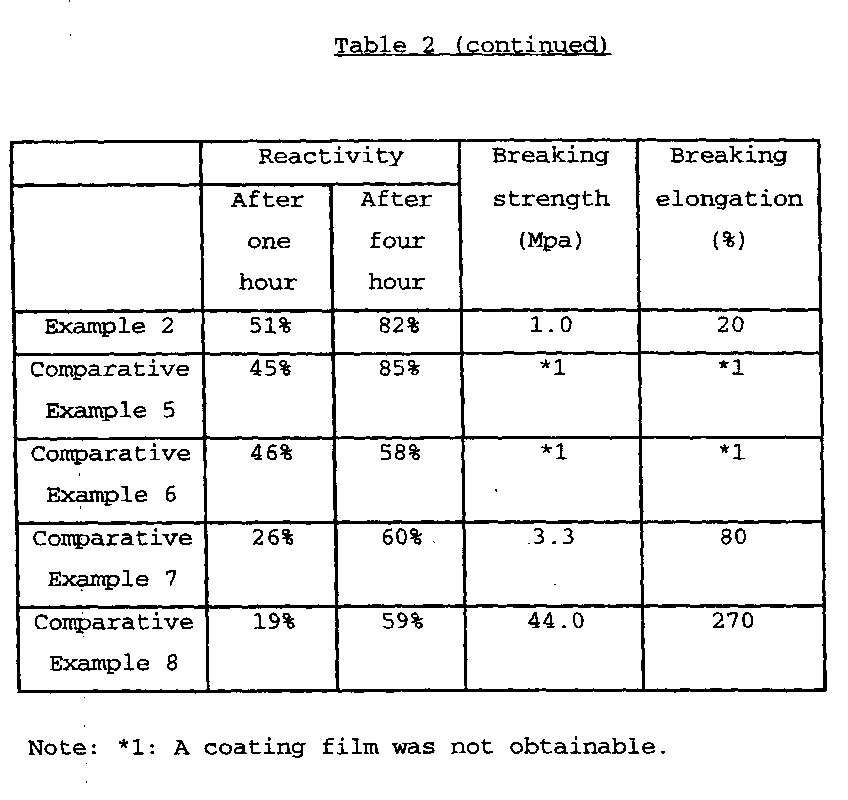

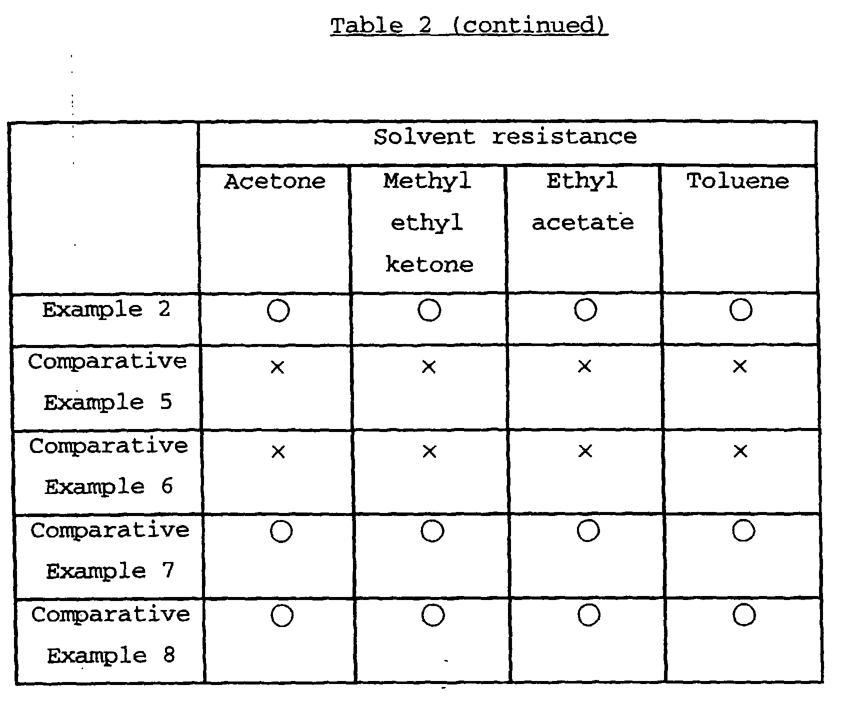

From the above Examples and Comparative Examples, the following facts were recognized.
(1) By comparing Example 1 with Comparative Examples 2 to 4, it was confirmed that although the viscosity of 3-isocyanate methyl-1,6-hexamethylene diisocyanate used in Example 1 was substantially identical to that of hexamethylene diisocyanate as a bifunctional compound, the NCO content of 3-isocyanate methyl-1,6-hexamethylene diisocyanate was larger than those of hexamethylene diisocyanate and isophorone diisocyanate as polyfunctional compounds.
(2) By comparing Example 2 with Comparative Examples 5 to 8, it was confirmed that the reactivity of 3-isocyanate methyl-1,6-hexamethylene diisocyanate used in Example 2 was identical to or higher than those of the bifunctional isocyanate compounds used in Comparative Examples 5 and 6, and higher than those of the polyfunctional isocyanate compounds used in Comparative Examples 7 and 8, and further the curability thereof was more excellent than those of these Comparative Examples.
(3) By comparing Example 2 with Comparative Examples 5 and 6, it was confirmed that in Example 2, the cured coating film was obtained, while in Comparative Examples 5 and 6, any cured coating film was not obtained.
(4) By comparing Example 2 with Comparative Examples 7 and 8, it was confirmed that although in Example 2, the amount of the isocyanate compound added was small, the solvent resistance of the obtained coating film was identical to those of Comparative Examples 7 and 8.

### Industrial Applicability

As is apparent from the above Examples, 3-isocyanate methyl-1,6-hexamethylene diisocyanate according to the present invention exhibits a low viscosity and, therefore, an excellent workability. In addition, the isocyanate compound according to the present invention have a high NCO content, so that even though the amount of the isocyanate compound added is smaller than those of the conventional isocyanate compounds, there can be obtained a coating film having similar properties. Further, 3-isocyanate methyl-1,6-hexamethylene diisocyanate according to the present invention has an excellent reactivity and a high curing speed, so that the cured product can show a high cross-linking density. Accordingly, the isocyanate compound according to the present invention, can be suitably applied to paints for foams, metals, plastics, woods, magnetic recording media, etc., and adhesives.

## Claims

1. 3-isocyanate methyl-1,6-hexamethylene diisocyanate.

2. A process for producing 3-isocyanate methyl-1,6-hexamethylene diisocyanate, comprising:
reacting 3-aminomethyl-1,6-hexamethylene diamine with phosgene in the presence of a tertiary amine.

3. A process for producing 3-aminomethyl-1,6-hexamethylene diisocyanate, comprising:
reacting 3-aminomethyl-1,6-hexamethylene diamine with phosgene in an inert solvent at a temperature of not more than 40°C in the presence of a tertiary amine to form a slurry containing tertiary amine hydrochloride as a precipitate;
successively heating said slurry to obtain a slurry containing 3-aminomethyl-1,6-hexamethylene diisocyanate;
filtering said slurry containing 3-aminomethyl-1,6-hexamethylene diisocyanate, thereby separating the slurry into a filter cake mainly containing tertiary amine hydrochloride and a filtrate mainly containing the inert solvent and 3-aminomethyl-1,6-hexamethylene diisocyanate; and
subjecting said filtrate to distillation in the presence of a hydrochloric acid scavenger, thereby obtaining 3-aminomethyl-1,6-hexamethylene diisocyanate.

4. A polyurethane resin which is produced by reacting 3-isocyanate methyl-1,6-hexamethylene diisocyanate with a polyol compound.

5. A paint which contains said polyurethane resin set forth in claim 4.

6. An adhesive which contains said polyurethane resin set forth in claim 4.
